# EUROPEAN PATENT SPECIFICATION

(11) **EP 0 529 528 B1**
(45) Date of publication and mention of the grant of the patent: **19.04.1995**
(21) Application number: 92114306.1
(22) Date of filing: 21.08.1992
(51) Int. Cl.: C07C 401/00, A61K 31/59

(54) **Novel vitamin D3 analogues**
Neue Vitamin-D3-Derivate
Nouveaux dérivés de vitamine D3

(30) Priority: 27.08.1991 US 750307
(43) Date of publication of application: 03.03.1993
(73) Proprietor: DAIKIN INDUSTRIES, LTD., Kita-ku, Osaka 530 (JP)
(72) Inventor: Kobayashi, Yoshiro, Tokyo-to (JP); Iseki, Katsuhiko, Tsukuba-shi, Ibaraki-ken (JP); Nagai, Takabumi, Tsukuba-shi, Ibaraki-ken (JP); Tanaka, Yoko, Delmar, N.Y. 12054 (US); Ikekawa, Nobuo, Musashino-shi, Tokyo-to (JP)
(74) Representative: Hansen, Bernd, Dr. Dipl.-Chem.

(56) References cited:
- EP-A- 0 296 800
- WO-A-89/10351
- CHEMICAL ABSTRACTS, vol. 102, no. 7, 18 February 1985, Columbus, Ohio, US; abstract no. 56746, H. KOEFFLER ET AL 'Induction of Macrophage Differentiation of Human Normal and Leukemic Myeloid Stem Cells by 1,25-Dihydroxyvitamin D3 and Its Fluorinated Analogs' page 125 ;column 1 ;
- JOURNAL OF MEDICINAL CHEMISTRY vol. 28, no. 9, September 1985, WASHINGTON US pages 1148 - 1153 S. YAMADA ET AL 'Syntheses and Differentiating Action of Vitamin D Endoperoxides. Singlet Oxygen Adducts of Vitamin D Derivatives in Human Myeloid Leukemia Cells (HL-60)'
- PROCEEDINGS OF THE NATIONAL ACADEMY OF SCIENCES OF USA vol. 84, no. 9, May 1987, WASHINGTON US pages 2610 - 2614 V. K. OSTREM ET AL '24- and 26-Homo-1,25-dihydroxyvitamin D3: Preferential Activity in Inducing Differentiation of Human Leukemia Cells HL-60 In Vitro '
- CHEMICAL AND PHARMACEUTICAL BULLETIN. vol. 40, no. 5, May 1992, TOKYO JP pages 1346 - 1348 K. ISEKI ET AL 'Synthesis of 24-Homo-26,26 ,26,27,27,27-hexafluoro-1.alpha.,22,25-tri hydroxyvitamin D3'

## Description

This invention relates to novel vitamin D₃ analogues having activity of calcium control in biobody and tumor cell differentiation-inducing activity.

### Prior art

It is known that a bio-metabolite of vitamin D₃, 1α,25-dihydroxyvitamin D₃ is called an "active-type vitamin D₃" and has the activity of promoting absorption of calcium via the intestinal tract and thereby is useful as a medicament for the treatment of bone diseases. Recently, it has been found that the active-type vitamin D₃ and analogues thereof have a differentiation-inducing activity for recovering normal cells from cancerous cells (cf. Hirobumi Tanaka et al., "Seikagaku" (Biochemistry), Vol. 55, 1323, 1983) and further that some of these compounds have a remarkable activity of inhibiting the progress of cancer (K.W. Colton et al., Lancet, Jan. 28, 188, 1989). It has, however, been known that these active-type vitamin D₃ compounds have high antagonistic activity against calcium metabolism which is undesirable side effect when used as an anti-tumor drug.

It is described in Japanese Patent First Publication (Kokai) No. 68528/1991 that novel vitamin D derivatives of the following formula have vitamin D-like activities. 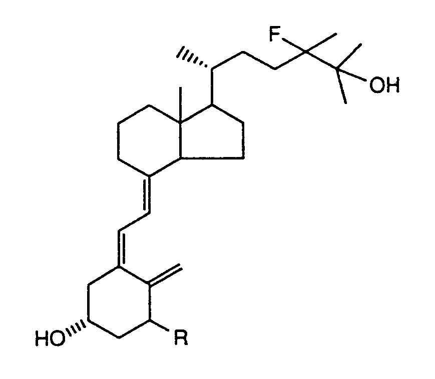
wherein R is hydrogen atom or hydroxy.

Furthermore, the article in PNAS-USA., VOL 84(9), pages 2610-2614 (1987) discloses a 24-homo Vitamin D₃ derivative with differentiation inducing activity.

### Summary Description of the Invention

The present inventors have intensively studied as to novel vitamin D₃ analogues which have excellent activities against calcium metabolism and cell differentiation.

An object of the invention is to provide novel vitamin D₃ analogues having pharmacological activities, especially anti-tumor activity owing to the cell differentiation-inducing activity. Another object of the invention is to provide a process for preparing the vitamin D₃ analogue. These and other objects and advantages of the invention will be apparent to the skilled persons in this field from the following description.

### Detailed Description of the Invention

The vitamin D₃ analogues of this invention have the following formula [I]: 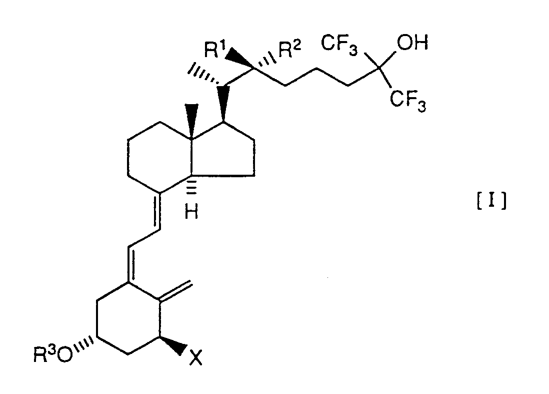
wherein R¹ is hydrogen atom and R² is hydroxy, or R¹ is hydroxy and R² is hydrogen atom, X is hydrogen atom, hydroxy or a hydroxy protected by a hydroxy-protecting group, and R³ is hydrogen atom or a hydroxy-protecting group.

In the present specification and claims, the hydroxy-protecting group includes a silyl ether type protecting group such as trimethylsilyl, t-butyldimethylsilyl, t-butyldiphenylsilyl, and the like.

Suitable examples of the compounds [I] are as follows.
26,26,26,27,27,27-Hexafluoro-24-homo-22S,25-dihydroxyvitamin D₃ (Compound A¹) [Compound [I] wherein R¹ = OH, R² = H, R³ = H, X = H]
26,26,26,27,27,27-Hexafluoro-24-homo-22R,25-dihydroxyvitamin D₃ (Compound A²) [Compound [I] wherein R¹ = H, R² = OH, R³ = H, X = H]
26,26,26,27,27,27-Hexafluoro-24-homo-1α,22S,25-trihydroxyvitamin D₃ (Compound B¹) [Compound [I] wherein R¹ = OH, R² = H, R³ = H, X = OH]
26,26,26,27,27,27-Hexafluoro-24-homo-1α,22R,25-trihydroxyvitamin D₃ (Compound B²) [Compound [I] wherein R¹ = H, R² = OH, R³ = H, X = OH]
3-Trimethylsilyl ether of Compound A¹ or A²
3-t-Butyldimethylsilyl ether of Compound A¹ or A²
3-t-Butyldiphenylsilyl ether of Compound A¹ or A²
1α,3-Bis(trimethylsilyl) ether of Compound B¹ or B²
1α,3-Bis(t-butyldimethylsilyl) ether of Compound B¹ or B²
1α,3-Bis(t-butyldiphenylsilyl) ether of Compound B¹ or B²
The compounds [I] of this invention can be prepared by various processes. One of the best processes is illustrated below.

A ketone compound of the formula [II]: 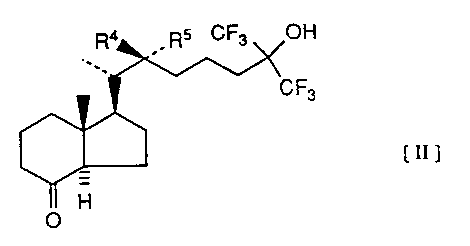
wherein R⁴ is a group of the formula: OR⁶ and R⁵ is hydrogen atom, or R⁵ is a group of the formula: OR⁶ and R⁴ is hydrogen atom, and R⁶ is a hydroxy-protecting group, is subjected to coupling reaction with an anion derived from a phosphine oxide of the formula [III]: 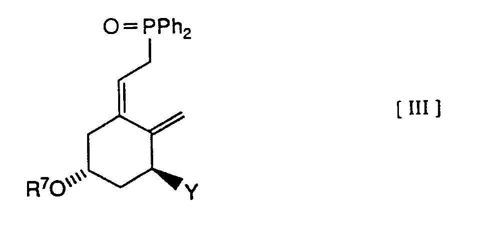
wherein R⁷ is a hydroxy-protecting group, Y is hydrogen atom or a group of the formula: OR⁷, and Ph means phenyl, optionally followed by removing the hydroxy-protecting group. The derivation of phosphine oxide to the anion is carried out in the presence of a base such as alkyllithium (e.g. n-butyllithium).

The above coupling reaction of the compound [II] and the compound [III] is usually carried out at a low temperature, for example -100°C to -50°C, preferably -80°C to -20°C, under an inert atmosphere (e.g. under argon gas) in an ether solvent (e.g. diethyl ether, tetrahydrofuran (THF), etc.) for 10 minutes to 24 hours, preferably for 30 minutes to 2 hours. The obtained product [I] can be purified by a conventional method, for example, by silica gel chromatography. The removal of the hydroxy-protecting group from the compound [I] can be carried out by a conventional method.

The hydroxy-protecting group R⁶ in the formula [II] is preferably a silyl group (e.g. t-butyldimethylsilyl, etc.) or an acyl group (e.g. acetyl, etc.). The hydroxy-protecting group R⁷ in the formula [III] is preferably a silyl group (e.g. t-butyldimethylsilyl etc.).

The starting compound [III] used in the above coupling reaction is prepared by a known process as disclosed in E.G. Baggiolini et al., J. Am. Chem. Soc., Vol. 104, 2945, 1982 and Japanese Patent First Publication (Kokai) No. 250844/1990.

On the other hand, the other starting compound [II] can be prepared by the process as illustrated by the following reaction scheme: 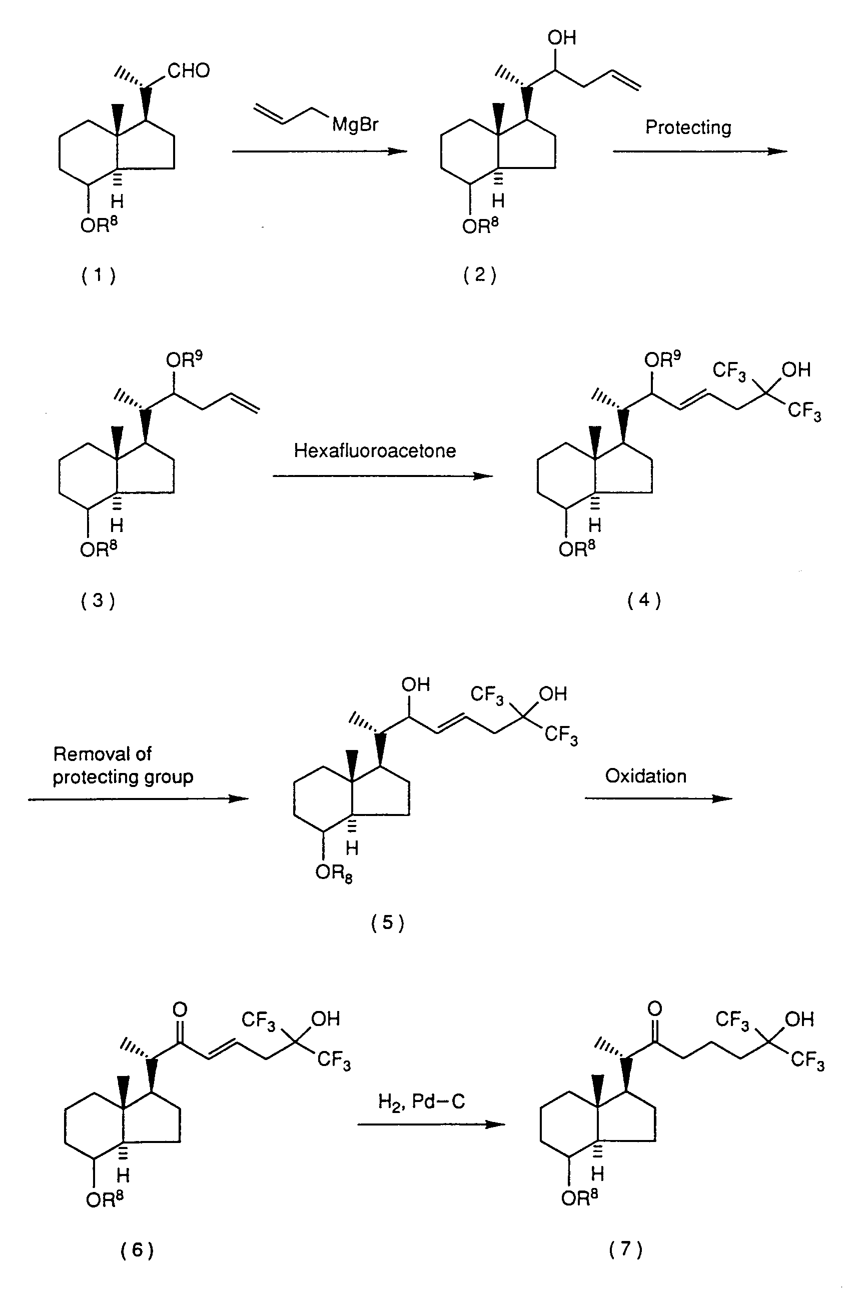 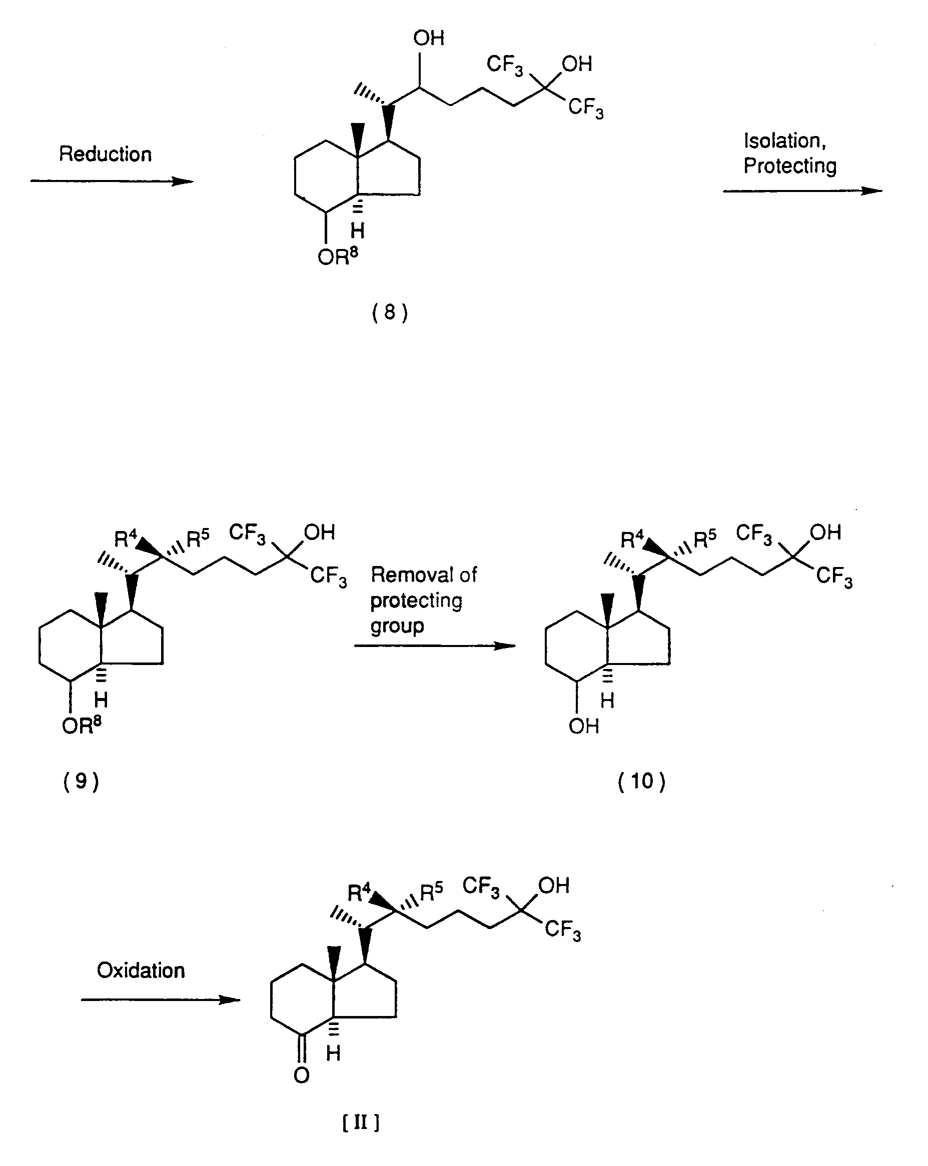
wherein R⁴ and R⁵ are as defined above, and R⁸ and R⁹ are each a hydroxy-protecting group.

According to the above process, the starting compound [II] can be prepared by reacting the aldehyde compound (1) with a Grignard reagent to give the compound (2), protecting the hydroxy group of the compound (2) with a hydroxy-protecting group in a usual manner, reacting the resultant compound (3) with hexafluoroacetone, removing the hydroxy-protecting group R⁹ from the resultant compound (4), oxidizing the resultant compound (5), hydrogenating the resultant compound (6) in the presence of palladiumcarbon, further reducing the resultant compound (7) to give compounds (8) which are mixture of two diastereomers: 22S isomer and 22R isomer, isolating each of the isomers of the compound (8) and then protecting the hydroxy group thereof, removing the hydroxy-protecting group R⁸ of the resultant compound (9), and finally oxidizing the compound (10). The detail conditions of the above process are illustrated by Reference Examples 1 to 10 hereinafter.

The compounds of this invention are illustrated by the following Examples and Reference Examples.

### Example 1

### 1-1. Preparation of 1α,3,22S-tris(t-butyldimethylsilyl) ether of 26,26,26,27,27,27-hexafluoro-24-homo-1α,22S,25-trihydroxyvitamin D₃ (Compound B¹) by Wittig reaction of compound [II] and compound [III]:

To a solution of a compound of the formula (III) wherein R⁷ is t-butyldimethylsilyl and Y is t-butyldimethylsilyloxy (320 mg) in anhydrous THF (8 ml) is added dropwise n-butyllithium (2.5 M, in the form of a solution in hexane) at -78°C, and the mixture is stirred at the same temperature for 5 minutes. To the mixture is added a solution of the 22S-compound (II) obtained in Reference Example 10 wherein R⁴ is t-butyldimethylsilyloxy and R⁵ is hydrogen atom (27 mg) in anhydrous THF (1 ml) at one time. The mixture is warmed to room temperature and then is stirred for 10 minutes. The reaction mixture is added to saturated aqueous ammonium chloride solution and is extracted with ethyl acetate. The ethyl acetate layer is washed with water and dried over anhydrous magnesium sulfate. After distilling off the solvent, the residue is purified by column chromatography (SiO₂, eluant, n-hexane-ethyl acetate= 20 : 1) to give the title compound (28.3 mg, 63.2 %) as colorless viscous substance.
¹H-NMR (CDCl₃) δ: 0.03 (3H, s), 0.06 (6H, s), 0.54 (3H, s), 0.83-0.93 (3H, m), 0.88 (9H, s), 0.88 (9H, s), 0.89 (9H, s), 1.12-2.08 (19H, m), 2.21 (1H, d-d, J=13.8, 7.4Hz), 2.45 (1H, d-d, J=13.8, 4.7Hz), 2.75-2.90 (1H, m), 3.02 (1H, bs), 3.55-3.77 (1H, m), 4.10-4.27 (1H, m), 4.33-4.45 (1H, m), 4.85-4.90 (1H, m), 5.17-5.23 (1H, m), 6.02 (1H, d, J=11.4Hz), 6.24 (1H, d, J=11.4Hz)
IR (neat): 3416, 2930 cm⁻¹

### 1-2. Preparation of 26,26,26,27,27,27-hexafluoro-24-homo-1α,22S,25-trihydroxyvitamin D₃ (Compound B¹) by removal of the protecting silyl group:

To a solution of the tris(t-butyldimethylsilyl) ether of Compound B¹ obtained in Example 1-1 (26.3 mg) in anhydrous THF (1 ml) is added dropwise a solution (0.3 ml) of tetra(n-butyl)ammonium fluoride in THF (1 M) at room temperature, and the mixture is stirred at room temperature for 12.5 hours. The reaction mixture is added to saturated aqueous ammonium chloride solution and is extracted with ethyl acetate. The organic layer is washed with water and dried over anhydrous magnesium sulfate. After distilling off the solvent, the residue is purified by column chromatography (SiO₂, eluant, n-hexane-ethyl acetate = 1 : 1) to give the Compound B¹ (15.58 mg, 95.8 %) as colorless needles.
¹H-NMR (CD₃OD) δ: 0.58 (3H, s), 0.92 (3H, d, J=6.5Hz), 1.20-2.35 (20H, m), 2.40-2.62 (1H, m), 2.75-2.95 (1H, m), 3.60-3.78 (1H, m), 4.40-4.25 (1H, m), 4.25-4.45 (1H, m), 4.57 (1H, bs), 4.95-5.00 (1H, m), 5.27-5.34 (1H, m), 6.09 (1H, d, J=11.2Hz), 6.33 (1H, d, J=11.2Hz)
IR (KBr): 3420, 2940 cm⁻¹

### Example 2

### Preparation of 26,26,26,27,27,27-hexafluoro-24-homo-22S,25-dihydroxyvitamin D₃ (Compound A¹):

In the same manner as described in Example 1 excepting that there is used a compound of the formula (III) wherein R⁷ is t-butyldimethylsilyl and Y is hydrogen atom, there is prepared Compound A (yield 62.0 %) as colorless powder.
¹H-NMR (CD₃OD) δ: 0.56 (3H, s), 0.91 (3H, d, J=6.7Hz), 1.20-2.60 (24H, m), 2.85 (1H, m), 3.65 (1H, m), 3.76 (1H, m), 4.74 (1H, bs), 5.03 (1H, bs), 6.03 (1H, d, J=11.0Hz), 6.22 (1H, d, J=11.0Hz)
IR (KBr): 3374, 2947, 1211, 1047 cm⁻¹

### Example 3

### 3-1. Preparation of 1α,3,22R-tris(t-butyldimethylsilyl) ether of 26,26,26,27,27,27-hexafluoro-24-homo-1α,22R,25-trihydroxyvitamin D₃ by Wittig reaction of compound [II] and compound [III]:

To a solution of a compound of the formula (III) wherein R⁷ is t-butyldimethylsilyl and Y is t-butyldimethylsilyloxy (452 mg) in anhydrous THF (11 ml) is added dropwise n-butyllithium (2.5 M, in the form of a solution in hexane) at -78°C, and the mixture is stirred at the same temperature for 5 minutes. To the mixture is added a solution of the 22R-compound (II) obtained in Reference Example 13 wherein R⁴ is hydrogen atom and R⁵ is t-butyldimethylsilyloxy (43.85 mg) in anhydrous THF (1.5 ml) at one time. The mixture is warmed to room temperature and then is stirred for 10 minutes. The reaction mixture is added to saturated aqueous ammonium chloride solution and is extracted with ethyl acetate. The ethyl acetate layer is washed with water and dried over anhydrous magnesium sulfate. After distilling off the solvent, the residue is purified by column chromatography (SiO₂, eluant, n-hexane-ethyl acetate= 20 : 1) to give the title compound (43.3 mg, 58.9 %) as colorless viscous substance.
¹H-NMR (CD₃OD) δ: 0.06 (3H, s), 0.09 (15H, s), 0.56 (3H, s), 0.86-1.05 (3H, m), 0.89 (9H, s), 0.90 (18H, s), 1.10-2.08 (20H, m), 2.15-2.29 (1H, m), 2.40-2.53 (1H, m), 2.77-2.92 (1H, m), 3.64-3.76 (1H, m), 4.18-4.30 (1H, m), 4.38-4.47 (1H, m), 4.82-4.90 (1H, m), 5.18-5.24 (1H, m), 6.07 (1H, d, J=11.4Hz), 6.25 (1H, d, J=11.4Hz)
IR (neat): 3421, 2929, 2857 cm⁻¹

### 3-2. Preparation of 26,26,26,27,27,27-hexafluoro-24-homo-1α,22R,25-trihydroxyvitamin D₃ (Compound B²) by removal of the protecting silyl group:

A solution of the tris(t-butyldimethylsilyl) ether of Compound B² obtained in Example 3-1 (43.3 mg) and acidic ion-exchange resin (50W-4x) (1.5 g) in methanol (15 ml) are stirred at room temperature for 24 hours. After the ion-exchange resin is filtered off, the filtrate is washed with ethyl acetate. After distilling off the solvent, the residue is purified by column chromatography (SiO₂, eluant, n-hexane-ethyl acetate = 1 : 1) to give the Compound B² (25.36 mg, 95 %) as colorless needles.
¹H-NMR (CD₃OD) δ: 0.59 (3H, s), 0.93 (3H, d, J=6.8Hz), 1.15-2.10 (20H, m), 2.20-2.33 (1H, m), 2.45-2.60 (1H, m), 2.80-2.93 (1H, m), 3.57-3.70 (1H, m), 4.07-4.22 (1H, m), 4.32-4.41 (1H, m), 4.85-5.00 (1H, m), 5.27-5.35 (1H, m), 6.10 (1H, d, J=11.4Hz), 6.32 (1H, d, J=11.4Hz)
IR (KBr): 3438, 2947, 1214 cm⁻¹

### Reference Example 1

### Preparation of Compound (2) wherein R⁸ is benzyl by subjecting Compound (1) to Grignard reaction:

To a solution of Compound (1) wherein R⁸ is benzyl (327 mg) in anhydrous diethyl ether (3 ml) is added dropwise allylmagnesium bromide-THF solution (2M, 2 ml) at 0°C, and the mixture is stirred for one hour. The reaction mixture is poured into saturated aqueous ammonium chloride solution and extracted with diethyl ether. The ether layer is washed with water and dried over anhydrous magnesium sulfate. After distilling off the solvent, the residue is purified by column chromatography ((SiO₂, eluant, n-hexane-methylene chloride = 1 : 1) to give two diastereomers, Compound (2-1) (176 mg, 47.2 %) and compound (2-2) (147 mg, 39.4 %).

The compound (2-1) has the following physical properties:
Colorless oil,
¹H-NMR (CDCl₃) δ: 0.92 (3H, d, J=5.9Hz), 0.97 (3H, s), 1.10-2.40 (16H, m), 3.64-3.80 (2H, m), 4.36 (1H, d, J=12.3Hz), 5.02-5.21 (2H, m), 5.64-5.87 (1H, m), 7.17-7.50 (5H, m),
IR (neat): 3421, 2938, 2865 cm⁻¹
The compound (2-2) has the following physical properties:
Colorless oil,
¹H-NMR (CDCl₃) δ: 0.93 (3H, d, J=6.8Hz), 0.99 (3H, s), 1.00-2.29 (16H, m), 3.64-3.78 (2H, m), 4.36 (1H, d, J=12.4Hz), 4.63 (1H, d, J=12.4Hz), 5.03-5.21 (2H, m), 5.66-6.00 (1H, m), 7.18-7.42 (5H, m),
IR (neat): 3406, 2937, 2864 cm⁻¹

### Reference Example 2

### Preparation of Compound (3) wherein R⁸ is benzyl and R⁹ is acetyl by acetylation of Compound (2):

A mixture of Compound (2-1) obtained in Reference Example 1 (176 mg), acetic anhydride (204 mg) and pyridine (1 ml) is stirred at room temperature for 13 hours. The reaction mixture is extracted with diethyl ether and the ether layer is washed with diluted hydrochloric acid, saturated sodium hydrogen carbonate solution, and saturated saline solution in this order, and dried over anhydrous magnesium sulfate. After distilling off the solvent, the residue is purified by column chromatography (SiO₂, eluant, n-hexane-methylene chloride = 2 : 1) to give Compound (3) (185 mg, 94.5 %).

The compound (3) has the following physical properties:
Colorless oil,
¹H-NMR (CDCl₃) δ: 0.96 (3H, s), 0.97 (3H, d, J=6.4Hz), 1.05-2.09 (14H, m), 2.03 (3H, s), 2.20 (1H, d-d-d, J=7.0, 7.0, 13.0Hz), 2.39 (1H, d-d-d, J=7.0, 7.0, 13.0Hz), 3.66-3.73 (1H, m), 4.35 (1H, d, J=12.3Hz), 4.61 (1H, d, J=12.3Hz), 4.97-5.14 (2H, m), 5.59-5.83 (1H, m), 7.17-7.38 (5H, m),
IR (neat): 2940, 2867, 1735 cm⁻¹

### Reference Example 3

### Preparation of Compound (4) wherein R⁸ is benzyl and R⁹ is acetyl by reacting Compound (3) with hexafluoroacetone:

To a solution of Compound (3) obtained in Reference Example 2 (1.20 g) in benzene (10 mg) is added hexafluoroacetone (1 ml) in a stainless steel autoclave, and the mixture is heated at 150°C for 39 hours. After allowing to cool, the reaction mixture is extracted with diethyl ether. After distilling off the solvent, the residue is purified by column chromatography (SiO₂, eluant, n-hexane-ethyl acetate = 10 : 1) to give Compound (4) (1.267 g, 73.7 %).

The compound (4) has the following physical properties:
Colorless needles,
M.p. 134.8 - 135.9°C,
¹H-NMR (CDCl₃) δ: 0.96 (3H, d, J=6.7Hz), 0.96 (3H, s), 1.08-2.11 (14H, m), 2.08 (3H, s), 2.71 (2H, d, J=7.2Hz), 3.41 (1H, s), 3.65-3.80 (1H, m), 4.35 (1H, d, J=12.3Hz), 4.61 (1H, d, J=12.3Hz), 5.50 (1H, d-t, J=15.6, 5.1Hz), 5.71 (1H, d-d, J=15.6, 5.2Hz), 7.19-7.40 (5H, m),
IR (KBr): 3364, 2932, 1720 cm⁻¹

### Reference Example 4

### Preparation of Compound (5) wherein R⁸ is benzyl by hydrolysis of Compound (4):

A solution of Compound (4) obtained in Reference Example 3 (1.195 g) and potassium carbonate (1.38 g) in methanol (15 ml) is stirred at room temperature for 48 hours. The reaction mixture is poured into water and is extracted with diethyl ether. The ether layer is washed with water and dried over anhydrous magnesium sulfate. After distilling off the solvent, the residue is purified by column chromatography (SiO₂, eluant, n-hexane-ethyl acetate = 2 : 1) to give Compound (5) (1.0186 g, 92.4 %).

The compound (5) has the following physical properties:
Colorless needles,
M.p. 130.0 - 130.5°C,
¹H-NMR (CDCl₃) δ: 0.87 (3H, d, J=6.4Hz), 0.97 (3H, s), 1.06-2.10 (14H, m), 2.73 (2H, d, J=7.5Hz), 3.26 (1H, bs), 3.69-3.78 (1H, m), 4.33 (1H, bs), 4.36 (1H, d, J=12.3Hz), 4.63 (1H, d, J=12.3Hz), 5.66 (1H, d-t, J=15.5, 7.5Hz), 5.79 (1H, d-d, J=15.5, 3.7Hz), 7.18-7.44 (5H, m),
IR (KBr): 3535, 3114, 2945 cm⁻¹

### Reference Example 5

### Preparation of Compound (6) wherein R⁸ is benzyl by oxidation of Compound (5):

To a mixture of pyridine (2.4 g) and anhydrous methylene chloride (15 ml) is added chromium trioxide (1.2 g) under argon gas at room temperature, and the mixture is stirred for 15 minutes. To the mixture is added Compound (5) obtained in Reference Example 4 under argon gas, and the mixture is stirred at room temperature for 10 minutes. The supernatant of the reaction mixture is taken by decantation, and the residue is washed with diethyl ether. The combined organic layer is washed with water, and dried over anhydrous magnesium sulfate. After distilling off the solvent, the residue is purified by column chromatography (SiO₂, eluant, n-hexane-ethyl acetate = 10 : 1) to give Compound (6) (963 mg, 96 %).

The compound (6) has the following physical properties:
Colorless oil,
¹H-NMR (CDCl₃) δ: 1.00 (3H, s), 1.11 (3H, d, J=6.9Hz), 1.00-2.10 (12H, m), 2.73 (1H, q-d, J=6.9, 9.9Hz), 2.86 (2H, d, J=7.6Hz), 3.68-3.78 (1H, m), 4.35 (1H, d, J=12.3Hz), 4.48 (1H, bs), 4.61 (1H, d, J=12.3Hz), 6.31 (1H, d, J=15.6Hz), 6.89 (1H, d-t, J=15.6, 7.6Hz), 7.20-7.43 (5H, m),
IR (neat): 3299, 2936, 1686, 1660 cm⁻¹

### Reference Example 6

### Preparation of Compound (7) wherein R⁸ is benzyl by reduction of Compound (6):

The Compound (6) obtained in Reference Example 5 (464 mg) is stirred under hydrogen gas in the presence of 5 % Pd-C catalyst (20 mg) in methanol (20 ml) for 1 hour. After removing the catalyst by filtration and distilling off the solvent, the residue is purified by column chromatography (SiO₂, eluant, n-hexane-ethyl acetate = 5 : 1) to give Compound (7) (446.7 mg, 96 %).

The compound (7) has the following physical properties:
Colorless oil,
¹H-NMR (CDCl₃) δ: 0.98 (3H, s), 1.09 (3H, d, J=6.8Hz), 1.05-2.12 (16H, m), 2.40-2.78 (4H, m), 3.68-3.77 (1H, m), 4.35 (1H, d, J=12.3Hz), 4.62 (1H, d, J=12.3Hz), 7.20-7.45 (5H, m),
IR (neat): 3283, 2936, 1694 cm⁻¹

### Reference Example 7

### Preparation of Compound (8) wherein R⁸ is benzyl by reduction of Compound (7):

To a solution of Compound (7) obtained in Reference Example 6 (446.7 mg) in ethanol (1 ml) is added sodium borohydride (33.4 mg), and the mixture is stirred at room temperature for 2.5 hours. The reaction mixture is poured into ice-water and extracted with diethyl ether and the ether layer is washed with water and dried over anhydrous magnesium sulfate. After distilling off the solvent, the residue is purified by column chromatography (SiO₂, eluant, methylene chloride-acetonitrile = 50 : 1) to give two diastereomers (22S and 22R) of Compound (8) (303 mg, 67 %, and 104 mg, 23 %, respectively).

The main product 22S-Compound (8) has the following physical properties:
Colorless needles,
M.p. 156.9 - 157.6°C,
¹H-NMR (CD₃COCD₃) δ: 0.91 (3H, d, J=6.5Hz), 0.97 (3H, s), 1.10-2.13 (19H, m), 3.24 (1H, d, J=5.6Hz), 3.60-3.80 (2H, m), 4.35 (1H, d, J=12.3Hz), 6.54 (1H, s), 7.14-7.45 (5H, m),
IR (KBr): 3536, 2942 cm⁻¹
The other isomer 22R-Compound (8) has the following physical properties:
Colorless needles,
M.p. 142.3 - 144.0°C,
¹H-NMR (CD₃CN) δ: 0.87 (3H, d, J=6.7Hz), 0.94 (3H, s), 1.00-2.10 (19H, m), 2.87 (1H, d, J=4.4Hz), 3.50-3.68 (1H, m), 3.68-3.80 (1H, m), 4.32 (1H, d, J=12.1Hz), 4.59 (1H, d, J=12.1Hz), 5.95 (1H, bs), 7.10-7.44 (5H, m),
IR (KBr): 3512, 3156, 2932 cm⁻¹

### Reference Example 8

### Preparation of 22S-Compound (9) wherein R⁴ is t-butyldimethylsilyloxy, R⁵ is hydrogen atom and R⁸ is benzyl by protecting 22S-Compound (8) with t-butyldimethylsilyl group:

To a solution of 22S-Compound (8) obtained in Reference Example 7 (164 mg) in anhydrous methylene chloride are added 2,6-lutidine (284 »l) and t-butyldimethylsilyl triflate (267 »l) at 0°C, and the mixture is stirred at room temperature for 13 hours. The reaction mixture is poured into ice-water and extracted with methylene chloride. The methylene chloride layer is washed with water and dried over anhydrous magnesium sulfate. After distilling off the solvent, the residue is purified by column chromatography (SiO₂, eluant, n-hexane-methylene chloride = 1 : 1) to give 22S-Compound (9) (200 mg, 100 %).

The 22S-Compound (9) has the following physical properties:
Colorless viscous substance,
¹H-NMR (CDCl₃) δ: 0.03 (3H, s), 0.05 (3H, s), 0.88 (9H, s), 0.88 (3H, d, J=5.7Hz), 0.96 (3H, s), 1.05-2.07 (20H, m), 3.05 (1H, s), 3.58-3.75 (2H, m), 4.36 (1H, d, J=12.4Hz, 4.62 (1H, d, J=12.4Hz), 7.19-7.43 (5H, m),
IR (neat): 3385, 2932, 2859 cm⁻¹

### Reference Example 9

### Preparation of 22S-Compound (10) wherein R⁴ is t-butyldimethylsilyloxy, and R⁵ is hydrogen atom by removal of benzyl group from 22S-Compound (9) by hydrolysis:

The 22S-Compound (9) obtained in Reference Example 8 (200 mg) is stirred under hydrogen gas in the presence of 5% Pd-C catalyst (10 mg) in methanol (10 ml) at room temperature and under atmospheric pressure for 13 hours. After removing the catalyst by filtration and concentrating the filtrate, the residue is purified by column chromatography (SiO₂, eluant, n-hexane-methylene chloride = 5 : 1) to give 22S-Compound (10) (162 mg, 95 %).

The 22S-compound (10) has the following physical properties:
Colorless needles,
M.p. 116.1 - 117.0°C,
¹H-NMR (CDCl₃) δ: 0.03 (3H, s), 0.04 (3H, s), 0.85 (3H, d, J=6.1Hz), 0.88 (9H, s), 0.92 (3H, s), 1.04-2.10 (20H, m), 3.56-3.71 (1H, m), 4.03-4.15 (1H, m), 4.27 (1H, bs),
IR (KBr): 3504, 2956, 1463 cm⁻¹

### Reference Example 10

### Preparation of 22S-Compound [II] wherein R⁴ is t-butyldimethylsilyloxy and R⁵ is hydrogen atom by oxidation of 22S-Compound (10):

A solution of 22S-Compound (10) obtained in Reference Example 9 (160 mg) in anhydrous methylene chloride is added dropwise to a suspension of pyridinium chlorochromate (216 mg) in anhydrous methylene chloride, and the mixture is stirred at room temperature for 2 hours. The reaction mixture is extracted with diethyl ether and the ether layer is washed with water and dried over anhydrous magnesium sulfate. After distilling off the solvent, the residue is purified by column chromatography (SiO₂, eluant, n-hexane-ethyl acetate = 5 : 1) to give 22S-Compound [II] (152 mg, 95 %).

The 22S-Compound [II] has the following physical properties:
Colorless needles,
M.p. 128.6 - 130°C,
¹H-NMR (CD₃OD) δ: 0.08 (3H, s), 0.09 (3H, s), 0.65 (3H, s), 0.91 (9H, s), 0.95 (3H, d, J=6.4Hz), 1.20-2.63 (20H, m), 3.66-3.78 (1H, m),
IR (KBr): 3243, 2961, 1698 cm⁻¹

### Reference Example 11

### Preparation of 22R-Compound (9) wherein R⁴ is hydrogen atom, R⁵ is t-butyldimethylsilyloxy and R⁸ is benzyl by protecting 22R-Compound (8) with t-butyldimethylsilyl group:

To a solution of 22R-Compound (8) obtained in Reference Example 7 (313 mg) in anhydrous methylene chloride are added 2,6-lutidine (284 »l) and t-butyldimethylsilyl triflate (267 »l) at 0°C, and the mixture is stirred at room temperature for 13 hours. The reaction mixture is poured into ice-water and extracted with methylene chloride. The methylene chloride layer is washed with water and dried over anhydrous magnesium sulfate. After distilling off the solvent, the residue is purified by column chromatography (SiO₂, eluant, n-hexane-methylene chloride = 1 : 1) to give 22R-Compound (9) (380 mg, 100 %).

The 22R-Compound (9) has the following physical properties:
Colorless viscous substance,
¹H-NMR (CDCl₃) δ: 0.03 (3H, s), 0.05 (3H, s), 0.88 (3H, d, J=6.8Hz), 0.88 (9H, s), 0.96 (3H, s), 0.81-2.09 (19H, m), 3.09 (1H, s), 3.58-3.76 (2H, m), 4.34 (1H, d, J=12.3Hz), 4.62 (1H, d, J=12.3Hz), 7.28-7.43 (5H, m),
IR (neat): 3422, 2932, 2860 cm⁻¹

### Reference Example 12

### Preparation of 22R-Compound (10) wherein R⁴ is hydrogen atom and R⁵ is t-butyldimethylsilyloxy by removal of benzyl group from 22R-Compound (9) by hydrolysis:

The 22R-Compound (9) obtained in Reference Example 11 (326.8 mg) is stirred under hydrogen gas in the presence of 5 % Pd-C catalyst (20 mg) in methanol (15 ml) at room temperature and under atmospheric pressure for 13 hour. After removing the catalyst by filtration and concentrating the filtrate, the residue is purified by column chromatography (SiO₂, eluant, n-hexane-methylene chloride = 5 : 1) to give 22R-Compound (10) (208 mg, 74 %).

The 22R-compound (10) has the following physical properties:
Colorless viscous substance,
¹H-NMR (CDCl₃) δ: 0.03 (3H, s), 0.04 (3H, s), 0.82-0.99 (3H, m), 0.89 (9H, s), 0.92 (3H, s), 0.99-2.12 (20H, m), 3.58-3.71 (1H, m), 4.04-4.19 (1H, m), 4.35 (1H, bs),
IR (neat): 3286, 2950, 1464 cm⁻¹

### Reference Example 13

### Preparation of 22R-Compound [II] wherein R⁴ is hydrogen atom and R⁵ is t-butyldimethylsilyloxy by oxidation of 22R-Compound (10):

A solution of 22R-Compound (10) obtained in Reference Example 12 (208 mg) in anhydrous methylene chloride is added dropwise to a suspension of pyridinium chlorochromate (260 mg) in anhydrous methylene chloride, and the mixture is stirred at room temperature for 4 hours. The reaction mixture is extracted with diethyl ether and the ether layer is washed with water and dried over anhydrous magnesium sulfate. After distilling off the solvent, the residue is purified by column chromatography (SiO₂, eluant, n-hexane-ethyl acetate = 5 : 1) to give 22R-Compound [II] (183 mg, 88 %).

The 22R-Compound [II] has the following physical properties:
Colorless needles,
M.p. 144 - 146°C,
¹H-NMR (CDCl₃) δ: 0.03 (6H, s), 0.64 (3H, s), 0.88 (9H, s), 0.94 (3H, d, J=6.7Hz), 1.17-2.50 (19H, m), 3.15 (1H, s), 3.58-3.69 (1H, m),
¹⁹F-NMR (CDCl₃) ppm; -76.80 (q, J=9.3Hz), -77.15 (q, J=9.3Hz),
IR (KBr): 3420, 2959, 2858, 1698 cm⁻¹

### Experiment 1

### Effects of the compounds on serum calcium concentration:

Method: In accordance with the method of Mori et al [Vitamin-gaku Jikken-ho (Experiments in Vitamin Science), [I], Fat Soluble Vitamins, Ed. by Japan Vitamin Association, issued by Tokyo Kagaku Dojin, pages 120-135], Vitamin D-defficient rats were prepared and the calcium concentration in serum was measured.

That is, Wistar rats (5 rats per each group) were fed with vitamin D-free, low calcium (0.02 %) feed for about 3 weeks.

After confirming the low calcium concentration in serum (less than 6 mg/dl), test compounds (650 pmole) solubilized in solvent (95 % propylene glycol + 5 % ethanol) were subcutanceously injected in the behind of the rats at a dose of 0.1 ml per day. In a control group, solvent only was injected likewise. Three and four days after the first injection, blood was taken and the calcium concentration in the serum was measured, and the averages of the obtained data are shown.

### Test compounds:

1. 1α,25-dihydroxy vitamin D₃
2. 26,26,26,27,27,27-hexafluoro-24-homo-1α,22S, 25-trihydroxyvitamin D₃ [Compound (I) of the present invention wherein R¹ = OH, R² = H, R³ = H, X = OH, which is the compound of Example 1-2 "Compound B¹"]
3. 26,26,26,27,27,27-hexafluoro-24-homo-1α,22R, 25-trihydroxyvitamin D₃ [Compound (I) of the present invention wherein R¹ = H, R² = OH, R³ = H, X = OH, which is the compound of Example 3-2 "Compound B²"]

Results: The test result are shown in the following Table 1.

**Table 1**

| Test compounds | Serum calcium concentration |
|---|---|
| None (control) | 4.5 mg/dl |
| 1α,25-diOH-vitamin D₃ | 6.9 mg/dl |
| Compound B¹ | 5.5 mg/dl |
| Compound B² | 5.6 mg/dl |

As is clear from the above test results, the compounds of the present invention showed less increase of the serum calcium concentration in comparison with the known 1α,25-dihydroxyvitamin D₃.

### Experiment 2

### Effects of the compound on differentiation-inducing activity

### Method:

Subculture cells (HT-29) derived from human colonic cancer were inoculated onto a 24-well plate for tissue culture and thereto was added calf serum in an amount of 10 % by weight, which was cultured in RPMI-1640 medium. After culturing for 24 hours, the supernatant was removed. To the residue was added a medium containing 2 x 10⁻³M sodium butyrate and 1α,25-dihydroxyvitamin D₃ or a vitamin D₃ analogue of this invention (exchange of the medium), and the mixture was subjected to station culture in a culture vessel containing carbon dioxide (5 % CO₂ - 95 % air) at 37°C. On every other day, the culture medium was exchanged with the same medium as mentioned above, and on the 7th day, the number of mucin-producing cells and the shape of the cells were observed by the method of Augeron et al. [cf. Cancer Res, Vol. 44, 3961, 1984].

It is known that the mucin production is observed in normal cells of the large intestine (the colon) but not in cancer cells. Accordingly, as a marker for measuring the fact that the cancer cells HT-29 had differentiated and could express characteristics of normal cells, the number of mucin-producing cells was measured.

Test compounds: The same as used in the above Experiment 1.

Results: The test results are shown in the following Table 2.

**Table 2**

| Test compounds | Concentration (M) | Number of mucin-producing cells (%) |
|---|---|---|
| None (control) | 0 | 22 |
| 1α,25-diOH-vitamin D₃ | 10⁻⁷ | 92 |
| | 10⁻⁸ | 48 |
| | 10⁻⁹ | 20 |
| Compound B¹ | 10⁻⁷ | 94 |
| | 10⁻⁸ | 93 |
| | 10⁻⁹ | 75 |
| Compound B² | 10⁻⁷ | 94 |
| | 10⁻⁸ | 69 |
| | 10⁻⁹ | 40 |

As is clear from the above test results, the compounds of the present invention showed increased number of mucin-producing cells, i.e. greater differentiation-inducing activity, in comparison with the known 1α,25-dihydroxyvitamin D₃.

## Claims

1. A vitamin D₃ analogue of the the formula [I]: 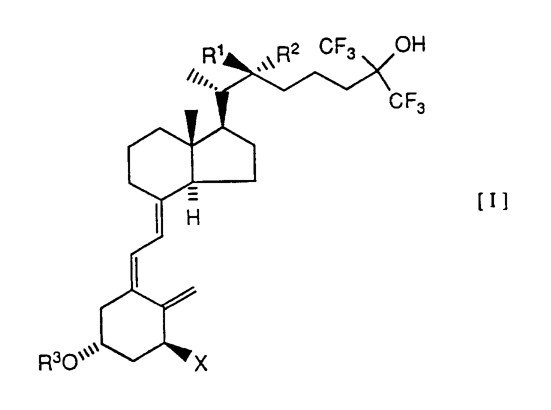 wherein R¹ is hydrogen atom and R² is hydroxy, or R¹ is hydroxy and R² is hydrogen atom, X is hydrogen atom, hydroxy or a hydroxy protected by a hydroxy-protecting group, and R³ is hydrogen atom or a hydroxy-protecting group.

2. The compound according to claim 1, wherein the hydroxy-protecting group is a member selected from the group consisting of trimethylsilyl, t-butyldimethylsilyl and t-butyldiphenylsilyl.

3. The compound according to claim 1 which is a member selected from the following groups:
26,26,26,27,27,27-hexafluoro-24-homo-1α,22S,25-trihydroxyvitamin D₃,
26,26,26,27,27,27-hexafluoro-24-homo-1α,22R,25-trihydroxyvitamin D₃,
1α,3-bis(trimethylsilyl) ether of 26,26,26,27,27,27-hexafluoro-24-homo-1α,22S,25-trihydroxyvitamin D₃,
1α,3-bis(trimethylsilyl) ether of 26,26,26,27,27,27-hexafluoro-24-homo-1α,22R,25-trihydroxyvitamin D₃,
1α,3-bis(t-butyldimethylsilyl) ether of 26,26,26,27,27,27-hexafluoro-24-homo-1α,22s,25-trihydroxyvitamin D₃,
1α,3-bis(t-butyldimethylsilyl) ether of 26,26,26,27,27,27-hexafluoro-24-homo-1α,22R,25-trihydroxyvitamin D₃,
1α,3-bis(t-butyldiphenylsilyl) ether of 26,26,26,27,27,27-hexafluoro-24-homo-1α,22S,25-trihydroxyvitamin D₃,
1α,3-bis(t-butyldiphenylsilyl) ether of 26,26,26,27,27,27-hexafluoro-24-homo-1α,22R,25-trihydroxyvitamin D₃.

## Patentansprüche

1. Vitamin D₃-Analogon der Formel [I]: 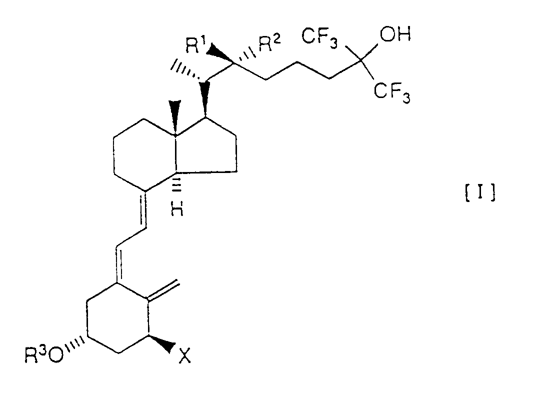 in der R¹ ein Wasserstoffatom und R² Hydroxy ist; oder R¹ Hydroxy und R² ein Wasserstoffatom ist; X ein Wasserstoffatom, Hydroxy oder ein durch eine Hydroxyschützende Gruppe geschütztes Hydroxy ist, und R³ ein Wasserstoffatom oder eine Hydroxy-schützende Gruppe ist.

2. Verbindung nach Anspruch 1, in der die Hydroxy-schützende Gruppe ein Glied, ausgewählt aus der aus Trimethylsilyl, t-Butyldimethylsilyl und t-Butyldiphenylsilyl bestehenden Gruppe, ist.

3. Verbindung nach Anspruch 1, welche ein Glied, ausgewählt aus den folgenden Gruppen, ist:
26,26,26,27,27,27-Hexafluor-24-homo-1α,22S,25-trihydroxyvitamin D₃,
26,26,26,27,27,27-Hexafluor-24-homo-1α,22R,25-trihydroxyvitamin D₃,
1α,3-Bis(trimethylsilyl)ether von 26,26,26,27,27,27-Hexafluor-24-homo-1α,22S,25-trihydroxyvitamin D₃,
1α,3-Bis(trimethylsilyl)ether von 26,26,26,27,27,27-Hexafluor-24-homo-1α,22R,25-trihydroxyvitamin D₃,
1α,3-Bis(t-butyldimethylsilyl)ether von 26,26,26,27,27,27-Hexafluor-24-homo-1α,22S,25-trihydroxyvitamin D₃,
1α,3-Bis(t-butyldimethylsilyl)ether von 26,26,26,27,27,27-Hexafluor-24-homo-1α,22R,25-trihydroxyvitamin D₃,
1α,3-Bis(t-butyldiphenylsilyl)ether von 26,26,26,27,27,27-Hexafluor-24-homo-1α,22S,25-trihydroxyvitamin D₃,
1α,3-Bis(t-butyldiphenylsilyl)ether von 26,26,26,27,27,27-Hexafluor-24-homo-1α,22R,25-trihydroxyvitamin D₃.

## Revendications

1. Analogue de vitamine D₃ de formule [I] : 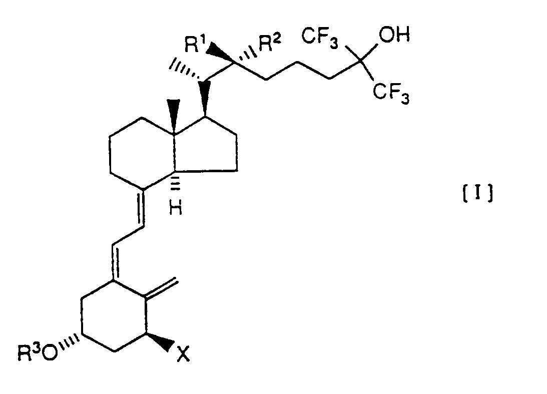 dans laquelle R¹ est un atome d'hydrogène et R² est un hydroxy, ou R¹ est un hydroxy et R² est un atome d'hydrogène, X est un atome d'hydrogène, un hydroxy ou un hydroxy protégé par un groupe protecteur d'hydroxy, et R³ est un atome d'hydrogène ou un groupe protecteur d'hydroxy.

2. Composé selon la revendication 1, dans lequel le groupe protecteur d'hydroxy est un membre choisi dans le groupe constitué du triméthylsilyle, du t-butyldiméthylsilyle et du t-butyldiphénylsilyle.

3. Composé selon la revendication 1 qui est un membre choisi dans les groupes suivants :
26,26,26,27,27,27-hexafluo-24-homo-1α,22S,25-trihydroxyvitamine D₃,
26,26,26,27,27,27-hexafluor-24-homo-1α,22R,25-trihydroxyvitamine D₃,
éther 1α,3-bis(triméthylsilylique) de 26,26,26,27,27,27-hexafluoro-24-homo-1α,22S,25-trihydroxyvitamine D₃,
éther 1α,3-bis(triméthylsilylique) de 26,26,26,27,27,27-hexafluoro-24-homo-1α,22R,25-trihydroxyvitamine D₃,
éther 1α,3-bis(t-butyldiméthylsilylique) de 26,26,26,27,27,27-hexafluoro-24-homo-1α,22S,25-trihydroxyvitamine D₃,
éther 1α,3-bis(t-butyldiméthylsilylique) de 26,26,26,27,27,27-hexafluoro-24-homo-1α,22R,25-trihydroxyvitamine D₃,
éther 1α,3-bis(t-butyldiphénylsilylique) de 26,26,26,27,27,27-hexafluoro-24-homo-1α,22S,25-trihydroxyvitamine D₃,
éther 1α,3-bis(t-butyldiphénylsilylique) de 26,26,26,27,27,27-hexafluoro-24-homo-1α,22R,25-trihydroxyvitamine D₃.
